# EUROPEAN PATENT APPLICATION

(11) **EP 4 524 984 A1**
(43) Date of publication of application: **19.03.2025**
(21) Application number: 23196865.2
(22) Date of filing: 12.09.2023
(51) Int. Cl.: G16H 20/70, G16H 20/40, G16H 40/63, A61B 5/055, A61M 21/02, A61B 5/00, G01R 33/28

(54) **SYSTEM FOR GENERATING PATIENT EXPERIENCE CONTENT DURING A MEDICAL PROCEDURE AND RELATED METHOD**

(71) Applicant: Koninklijke Philips N.V., 5656 AG Eindhoven (NL)
(72) Inventor: BUIL, Vincentius Paulus, Eindhoven (NL); BULUT, Murtaza, Eindhoven (NL); VAN EE, Raymond, 5656AG Eindhoven (NL); BEREZHNOY, Igor, Eindhoven (NL); SPELT, Hanne Adriana Alijda, Eindhoven (NL)
(74) Representative: Philips Intellectual Property & Standards

(57) **Abstract**

The invention relates to a system (6) for generating patient experience content (25) during a medical procedure performed by a medical device (2) in a medical environment (1). The system (6) comprises an input unit (7), a computing unit (8) and an output unit (9). The input unit (7) is adapted to receive sound information (15) about environment sounds produced in the medical environment (1), more particularly about equipment sounds produced by the medical device (2). The computing unit (8) is adapted to generate the patient experience content (25) relating to the environment sounds based on the sound information (15) and the output unit (9) is adapted to output the generated patient experience content (25). The invention further relates to a medical apparatus comprising said system (6) and to a related method (13).

## Description

### FIELD OF THE INVENTION

The invention relates to medical procedures and, in particular, to a system for generating patient experience content during a medical procedure that is performed by a medical device. The invention further relates to a medical apparatus comprising said system and to a related method.

### BACKGROUND OF THE INVENTION

Patients may experience a medical procedure, e.g., a medical imaging procedure, as worrying, frightening, and/or uncomfortable, for example, because of being confined to a small space such as a bore. In order to improve this experience, videos may be shown to patients in order to distract them. However, these videos do not take account of, sometimes loud, sounds produced by the medical equipment. These sounds may delude the experience, startle, worry and/or overstimulate the patient, which may lead, e.g., to patient movement and/or a change in vital signs, such as respiration and/or heart rhythm, and consequently longer exam times and/or lower quality medical images.

### SUMMARY OF THE INVENTION

It is therefore an object of the present invention to provide an improved experience to patients, in particular to provide a system for generating patient experience content during a medical procedure that at least partially overcomes the above-mentioned problems. It is a further object of the present invention to provide a medical apparatus with said system and to provide a related method. The object of the present invention is solved by the subject matter of the independent claims, wherein further embodiments are incorporated in the dependent claims.

In an aspect of the present invention, a system for generating patient experience content during a medical procedure is provided.

In this context, patient experience content is any stimulus that may be perceived by the patient, both consciously and unconsciously.

The medical procedure is performed by a medical device.

The medical device is in a medical environment, wherein said medical environment may comprise the equipment and the accessories needed for the medical procedure.

The system comprises an input unit, a computing unit and an output unit.

The input unit is adapted to receive sound information about environment sounds produced in the medical environment. In particular, said environment sounds may be equipment sounds produced by the medical device. Further, the environment sounds may comprise sounds produced by clinicians, e.g., as they talk and/or move around, and/or sounds produced by other equipment in the medical environment, such as other medical devices, alarms, or cutting equipment. In other words, the environment sounds may comprise all sounds that may be perceived by a patient during the medical procedure. The sound information may further comprise information about silence, i.e., the absence of any sounds that the patient may perceive.

The computing unit is adapted to generate the patient experience content relating to the environment sounds based on the sound information and the output unit is adapted to output the generated patient experience content. In particular, the computing unit may comprise one or more processors and may be a computing unit dedicated to the system or may be a computing unit of the medical device. The computing unit may also be referred to as patient experience rendering engine. The output unit may comprise an interface to which output devices such as screens, projectors, speakers, or other sensory stimulation device, e.g., temperature stimulation devices, air flow device, or tactile stimulation devices, may be connected. The patient experience content relates to the environment sounds to make the medical procedure more comforting for the patient and to strengthen the experience of the patient.

Since the patient experience content relates to the environment sounds, it may help the patient to relax and lower the patient's anxiety during the medical procedure. This may lead to less movement of the patient and hence to shorter exam times, improved medical procedure, e.g., better quality of the medical procedure, and/or reduced involvement of the medical staff.

According to an embodiment, the medical device is a medical imaging device. In this case, the medical imaging environment may be a medical imaging room or a medical imaging station. The medical procedure is then a medical imaging procedure and may include medical imaging scans as well as preparation steps and wrap-up steps. Examples for such a medical imaging device are a magnetic resonance imaging (MRI) device, a computed tomography (CT) imaging device, or an image guided therapy (IGT) imaging device. Alternatively, or additionally, the medical device is a medical therapy device, such as an IGT therapy device, or a radiotherapy device. These devices may produce equipment sounds that may worry or startle a patient and hence the patient experience content will be beneficial for the patient when a medical procedure is performed by any of these medical devices. Other medical devices may be, e.g., ventilators, dialysis equipment or patient monitors, i.e., patient alarms.

According to an embodiment, the sound information is received by getting operation signals from the medical device and obtaining the sound information based on said operation signals. In this context, operation signals may be any signals relating to the operation of the medical device, e.g., starting a unit, running a unit, stopping a unit, including parameters of these processes, closing or opening a switch, etc. The operation signals may correspond to the triggering of events in an application, e.g., a software application, such as a flag update, a variable update, a function execution, data retrieval, etc. In particular, the operation signals may be parameters of a scan sequence of a medical imaging procedure. In this case, the sound information may be obtained in real-time or may even be predicted when the scan sequences are planned in advance. The sound information may be obtained based on the operation signals via a library. Said library may link the operation signals to equipment sounds, to which sound recognition is applied. In particular, the library may comprise a recorded equipment sound for every operation signal. Alternatively, or additionally, the library may link the operation signals directly to sound information, wherein the library comprises sound information for every operation signal. Alternatively, or additionally, the sound information may be obtained based on the operation signals via a processor that translates the operation signals into equipment sounds. As an example, the processor may use an imaging protocol to estimate sound generation properties and then calculate the equipment sounds and/or the sound information using a sound database.

Alternatively, or additionally, the input unit is adapted to receive the sound information by monitoring the environment sounds via sound sensors and applying sound recognition on the monitored sounds. In particular, said sound sensors may be microphones. However, other sound sensors such as accelerometers measuring the acceleration of surfaces, may also be used. For monitoring the environment sounds, the microphones may be placed close to the patient, more particularly close to the patient's head, in order to monitor the sounds that the patient actually hears and/or feels. Both monitoring the environment sounds and applying the sound recognition may be performed in real-time such that the patient may receive the patient experience content that relates to the current environment sounds. Sound recognition may comprise the extraction of sound features, sound generation features and/or sound characteristics from the monitored environment sounds. Said sound recognition may be performed in the time domain and/or the frequency domain and may be performed with deterministic algorithms and/or with artificial intelligence, e.g., with trained neural networks. In particular, the sound recognition may also be referred to as sound feature calculation.

According to an embodiment, the sound information comprises sound features and/or sound generation features. The sound features comprise at least one out of timing, rhythm, beats per minute, pitch, loudness, frequency spectrum, energy in specific frequency bands, tempo, beat duration, and spectral features. The sound generation features may comprise start time, end time, duration of the sound, and sound continuance features, wherein the sound continuance features comprise information on whether the sound is starting, continuing or ending. Hence, the sound information comprises quantifiable features that are also relevant to the patient's perception of the sounds.

According to an embodiment, the patient experience content comprises visual content, auditory content, tactile stimulus content, vestibular stimulus content, thermoception content and/or air flow content. The visual content may comprise video content, images, different brightness and/or color. In particular, video content and/or images may be presented to the patient via a side-wall imagery projection, an in-bore imagery projection, or an in-bore mirrored projection. The visual content may also be presented via a head-mounted display, e.g., in the form of a virtual, augmented, or mixed reality experience. The auditory content may be presented to the patient via speakers or headphones. Tactile content may be presented to the patient via moving elements that are, e.g., integrated in a patient table or a topper on the patient table. The vestibular stimulus content may be electrical and/or galvanic vestibular stimulus content and may be presented to the patient, e.g., via electrodes attached to the patient. The thermoception content may be provided, e.g., using heating and/or cooling elements. The air flow content may comprise air events such as air puffs or air blowing. In addition to the above mentioned patient experience contents, further content may be presented to the patient, e.g., olfactory content using fragrances. In any case, the patient experience content is perceived by the patient and, as the patient experience content relates to the environment sounds that are also perceived by the patient, a more enjoyable sensory perception is provided to the patient.

According to an embodiment, the computing unit is adapted to generate the patient experience content such that it is congruent, synchronized and/or linked with the environment sounds. In other words, the patient experience content relates to the environment sounds, in particular, such that the patient perceives the environment sounds as belonging to an overall theme that includes the environment sounds and the patient experience content. This interplay of the patient experience content with the environment sounds serves to entertain the patient and hence to calm down the patient, distract him or her from the medical procedure, reduce worry, fears and/or discomfort, and/or reduce the risk of overstimulation.

According to an embodiment, the patient experience content relates to at least one motion related element. The motion of this motion related element then relates to the environment sound such that changes in the sound may correspond to changes in the motion of the motion related element. As an example, the motion related element may be an animated character and the motion of this animated character corresponds to the environment sound, in particular, the movements of the animated character are synchronized with the environment sound. As another example, the motion related element may comprise video scenes that correspond to the environment sound. As yet another example, the motion related element may be a changing theme feature, such that the brightness, color, images and/or types of objects in said theme feature change depending on the environment sound. Further patient experience content that is related to said motion related element may also be presented to the patient, such as audio content, olfactory content, haptic content, tactile content, vestibular content, temperature content. This further patient experience content may enhance the perception of the motion of the at least one motion related element.

According to an embodiment, the computing unit comprises a character and/or object animation rendering engine. Said character and/or object animation rendering engine may be similar to a character and/or object animation rendering engine used in video games, where character and/or object animations are also generated in real-time. The character and/or object animation rendering engine may comprise a library, in particular a library containing character and/or object animations. The character and/or object animations are then rendered in synchrony to the environment sounds to create a congruent, integrated experience for the patient. The character and/or object animation selection depends on the environment sounds and may further depend on the currently presented patient experience content, in order to create a continuous story and/or match a current phase in a storyline. Alternatively, or additionally, the character and/or object animation rendering engine may comprise a function that translates the sound information into character and/or object animations. Said function may relate, e.g., certain aspects of the sound information to corresponding movements of the character and/or object.

According to an embodiment, the computing unit is further adapted to select a patient experience content theme, wherein a patient experience content theme may be related to a specific animated character, or to a specific genre of videos or images. The patient experience content theme may be selected from a theme database. If it is foreseeable which environment sounds will be encountered during the medical procedure, the theme may be chosen depending on whether said theme comprises patient experience content for most of or for all of the expected environment sounds. Hence, the patient experience content theme may be chosen based on the available animation options for the patient experience content theme. When the patient experience content theme has been chosen and when it is known which equipment sounds will be produced, character and/or object animations may be planned ahead. In particular, a selected character and/or object may already appear in the patient experience content and can then start moving when the equipment sounds are actually perceived by the patient. Alternatively, or additionally, the patient experience content theme may be chosen based on a patient profile which, e.g., includes the patient's preferences. Hence, the patient experience during the medical procedure is further improved.

According to an embodiment, the computing unit is further adapted to include further visual content, auditory content and/or game content in the patient experience content. In this context, the "further" content relates to content that is not directly related to the environment sounds, but provides additional distraction and/or comforting to the patient and hence, improves the overall patient experience during the medical procedure.

According to an embodiment, the computing unit is adapted to generate the auditory content and/or the visual content by combining audio segments and/or video segments from an archive. The audio segments and/or video segments may also be referred to as audio loops and/or video loops, respectively. Said audio segments and/or video segments in the archive may be labeled according to at least some of the sound information that they correspond to. As an example, the audio segments and/or video segments may be labeled based on a value of beats per minutes that they correspond to and/or based on a volume that they correspond to. When a sound information with certain beats per minute or a certain volume is received, the corresponding audio and/or video segments will be chosen. This is an easy-to-implement way of generating the patient experience content.

According to another aspect of the present invention, a medical apparatus is provided. The medical apparatus comprises a medical device, a system for generating patient experience content and at least one output device. The medical apparatus may be a medical imaging apparatus and the medical device may be a medical imaging device, such as a magnetic resonance imaging (MRI) device, a computed tomography (CT) imaging device, or an image guided therapy (IGT) imaging. Alternatively, or additionally, the medical apparatus may be a medical therapy apparatus and the medical device may be an IGT therapy device, or a radiotherapy device. The system for generating patient experience content is that of the above description. In particular, any of the above-described embodiments or combinations thereof may be used as the system for generating patient experience content. Also, the advantages described above apply similarly to the medical apparatus.

The at least one output device is adapted to receive the generated patient experience content and provide said patient experience content to a patient. In particular, the at least one output device receives the patient experience content via an interface of the system for generating patient experience content, via a wired and/or a wireless connection. For visual content, the output device may be a screen, a projector, or a head-mounted display. For auditory content, the output device may be a speaker or headphones. For tactile stimulus content, the output device may be a moveable element and for vestibular stimulus content, the output device may be an electrode.

According to yet another aspect of the present invention, a method for generating patient experience content during a medical procedure performed by a medical device is provided. In particular, said method may be performed by the system for generating patient experience content according to the above description.

The medical device may be a medical imaging device such as a magnetic resonance imaging (MRI) device, a computed tomography (CT) imaging device, or an image guided therapy (IGT) imaging device, or a medical therapy device such as an IGT therapy device, or a radiotherapy device. Said medical device is in the medical environment, wherein said medical environment may comprise the equipment and the accessories needed for the medical procedure. As an example, the medical environment may be a medical imaging environment such as a medical imaging room or a medical imaging station.

According to the method, sound information about environment sounds produced in the medical environment is received. In particular, said environment sounds may be equipment sounds produced by the medical device. Further, the environment sounds may comprise sounds produced by clinicians, e.g., as they talk and/or move around, and/or sounds produced by other equipment in the medical environment, such as other medical devices, alarms, or cutting equipment. In other words, the environment sounds may comprise all sounds that may be perceived by a patient during the medical procedure. The sound information may further comprise information about silence, i.e., the absence of any sounds that the patient may perceive.

Then, patient experience content relating to the environment sounds is generated based on the sound information. The patient experience content relates to the environment sounds to make the medical procedure more comforting for the patient and to strengthen the experience of the patient. The patient experience content is then output and provided to the patient. Since the patient experience content relates to the environment sounds, it may help the patient to relax and lower the patient's anxiety during the medical procedure. This may lead to less movement of the patient and hence to shorter exam times and/or better quality of the medical procedure.

According to an embodiment, the method further comprises monitoring an effect of the patient experience content onto a state of the patient. The patient experience content is then generated also based on the monitored effect onto the state of the patient. The state of the patient may be a mental state and/or a physiological state. The monitoring may be performed in real-time and may be performed by analyzing facial expressions, a heart rhythm of the patient, muscle tension, a breathing rate, breathing patterns, motion of body parts of the patient, skin temperature, sweating, electrodermal activity, electrical activity of the brain, and/or blood pressure. The monitoring of the state of the patient may be performed, e.g., with cameras, with pressure sensors and/or with other sensors. To generate the patient experience content based on the state of the patient, the patient experience content may be labeled accordingly, e.g., as more calming or as more distracting. Hence, the patient experience is further improved.

According to an embodiment, the patient experience content is generated also based on a patient profile. Said patient profile may comprise personal data, a medical profile, information on previous experiences of the patient, information on patient sensitivity, e.g., to pain, temperature, and/or fear, and/or patient preferences. The information on previous experiences may comprise, e.g., previous responses to patient experience content. Further, the patient preferences may be obtained, e.g., using a questionnaire. Using said patient profile to generate the patient experience content, the patient experience may be further improved.

It shall be understood that a preferred embodiment of the invention can also be any combination of the dependent claims with the respective independent claim. Further, it has to be noted that embodiments of the invention are described with reference to different subject matters. In particular, some embodiments are described with reference to device type claims whereas other embodiments are described with reference to method type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features. These and other aspects of the invention will be apparent from and elucidated with reference to the embodiments described hereinafter.

### BRIEF DESCRIPTION OF THE DRAWINGS

In the following, preferred embodiments of the invention will be described, by way of example only, and with reference to the drawings in which:
Fig. 1 shows a schematic view of a medical imaging environment with a medical imaging device and an embodiment of a system for generating patient experience content;
Fig. 2 shows a schematic view of a medical imaging environment with a medical imaging device and another embodiment of a system for generating patient experience content; and
Fig. 3 shows a flowchart of an embodiment of a method for generating patient experience content.

In the figures, elements which correspond to elements already described may have the same reference numerals. Examples, embodiments or optional features, whether indicated as non-limiting or not, are not to be understood as limiting the invention as claimed.

### DETAILED DESCRIPTION OF EMBODIMENTS

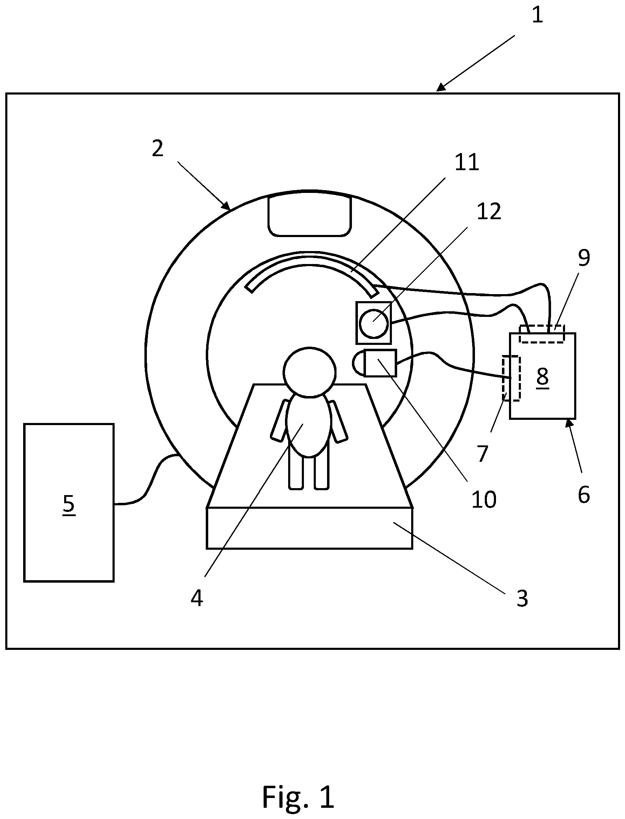

Fig. 1 shows a medical environment 1 with a medical device 2. In particular, a medical imaging environment with a medical imaging device is shown. The medical imaging device may be a magnetic resonance imaging (MRI) device, a computed tomography (CT) imaging device, or an image guided therapy (IGT) imaging device. However, the invention also applies to other medical devices such as medical therapy devices, e.g., IGT therapy devices, or radiotherapy devices, ventilators, dialysis equipment or patient monitors. The invention further applies to the environments corresponding to said medical devices, such as an MRI room, a CT room, an IGT lab, an intensive care unit (ICU), and/or a recovery room. The medical device 2 comprises a patient table 3 on which a patient 4 is shown. The medical device 2 also comprises a control unit 5 adapted to control the medical device 2. The medical environment 1 may further comprise other medical devices, auxiliary devices and/or staff, such as clinicians.

Further shown is a system 6 for generating patient experience content. Said system 6 comprises an input unit 7, shown as an input interface, a computing unit 8 and an output unit 9, shown as an output interface.

The input unit 7 is adapted to receive sound information about environment sounds produced in the medical environment, especially about equipment sounds produced by the medical device 2. In the present embodiment, said environment sounds are monitored via a microphone 10 that is connected to the input unit 7. Sound recognition is then applied to said monitored environment sounds in order to extract the sound information.

Based on the sound information, the computing unit 8 generates the patient experience content relating to the environment sounds. The patient experience content is then output by the output unit 9. In the current embodiment, the patient experience content is output to a screen 11 and a speaker 12, however, other output devices like projectors or moveable elements are also possible.

Since the patient experience content relates to the environment sounds, it may help the patient 4 to relax and lower the patient's anxiety during the medical procedure. This may lead to less movement of the patient 4 and hence to shorter exam times, better quality of the medical procedure and/or less involvement or distraction of the medical staff.

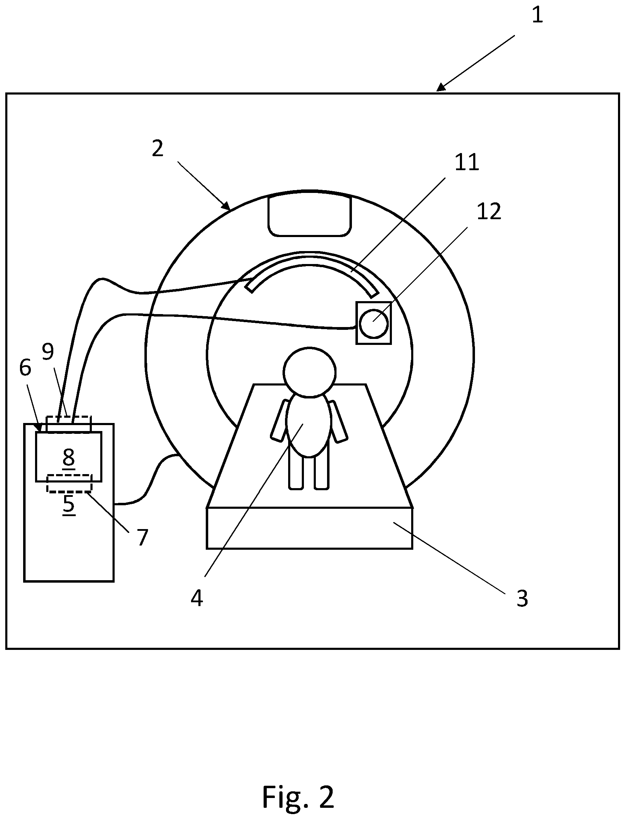

Fig. 2 shows a medical environment 1 with a medical device 2 and another embodiment of a system 6 for generating patient experience content. In this embodiment, the system 6 for generating patient experience content is a part of the control unit 5 of the medical device 2. The computing unit 8 may be separate from a main computing unit of the control unit 5, but may also be integrated in the main computing unit of the control unit 5.

The input unit 7 in this embodiment is adapted to receive operation signals from the medical device 2. That is, in this example, the input unit 7 is an internal unit within the control unit 5 of the medical device 2 and may even be an abstract unit. Based on the received operation signals, the sound information is obtained, e.g., via a library and/or via a processor that translates the operation signals into equipment sounds that can then be analyzed via sound recognition. Hence, in this embodiment, a microphone 10 is not needed. However, this embodiment can be combined with the previous embodiment, i.e., both operation signals can be obtained and environment sounds can be monitored via microphones 10, to achieve even better results.

The computing unit 8 then generates the patient experience content, which is then output by the output unit 9 to the output devices, i.e., the screen 11 and the speaker 12.

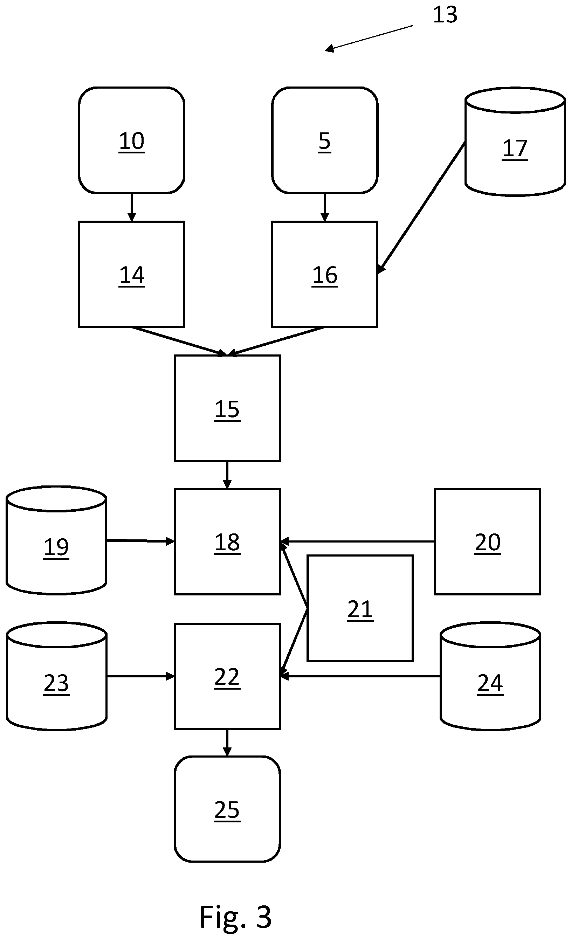

Fig. 3 shows a flowchart of a method 13 for generating patient experience content.

According to the method 13, environment sounds are captured by the microphone 10, and sound recognition 14 is applied to said environment sounds to extract sound information 15. Said sound information 15 may comprise the environment sounds, sound features and/or sound generation features.

Alternatively, or additionally, operation signals are received from the control unit 5 of the medical device 2 and the sound information 15 is obtained 16 via a library 17 with equipment sounds and/or via a processor that translates the operation signals into equipment sounds.

Then, the computing unit 8 generates 18 motion related content based on the sound information 15. For this, a library of character animations 19 may be used. Also, a state 20, in particular a mental state or a physiological state, of the patient 4 may be monitored, and information on said state 20 may be used to improve the motion related content. Further, information from a patient profile 21 may be used to improve the motion related content.

Moreover, the computing unit 8 adds 22 further content to the motion related content. As an example, audio content, video content and/or game content may be added from a library 23. Alternatively, or additionally, the additional content may come from a story engine or a game engine 24. Further, information from the patient profile 21 may be used to refine said additional content.

The complete content is the output as patient experience content 25. As described above, the patient experience content 25 is sent to output devices such as a screen 11 or a speaker 12.

Since the patient experience content 25 relates to the environment sounds, it may help the patient 4 to relax and lower the patient's anxiety during the medical procedure. This may lead to less movement of the patient 4 and hence to shorter exam times and/or better quality of the medical procedure.
the following, some additional examples are presented.

As an example, in addition to presenting a visual stimulus and an auditory stimulus, also a tactile and/or vestibular stimulus may be presented to the patient 4, wherein the stimulus has a variation in rhythm that matches the rhythm of the equipment sounds. This may help the patient 4 to focus on the sensory stimulation, thereby facilitating relaxation, focus, as well as a modification in breathing rate.

As another example, the synchronization between the environment sounds and the state 20 of the patient 4 may be further used to determine the stress/anxiety level of that patient 4, and to adapt the patient experience content 25 accordingly. By synchronizing a sound trace and a physiological trace, the impact that the sound has on a patient's 4 physiological system may be determined, as this will vary per person. This can be done either at the beginning of the medical procedure or before the medical procedure. Once it is known how much of the variability in physiological levels is caused by sound, it can be inferred that the additional variability in physiological levels may be caused by an affective state. As an example, higher arousal than expected may link to an affective state such as anxiety or stress. Based on an estimated affective state, the system may trigger certain calming interventions such as a short break with paced breathing, some reassuring instructions of the staff or adapting other aspects of the patient experience content 25 towards a more calming environment. Thus, the patient experience content 25 may be chosen dependent on the measured physiological levels representative of anxiety, while also controlling for other factors that may dilute the representation of anxiety in physiology.

As yet another example, the estimated state 20 of the patient 4, in particular the estimated psychological state of the patient 4 may be used to determine the patient experience content 25. For example, if the patient 4 is not bothered by sounds produced by an MRI device, having content synchronized with the equipment sounds can actually trigger the patient 4 to focus on the sounds and can start annoying the patient 4. Or if the patient 4 is too anxious, having synchronized patient experience content 25 can exacerbate the annoyance. In such a situation, it may be beneficial to purposefully create asynchrony between the equipment sounds and the patient experience content 25, using the same system. To account for prior information related to perception of sounds, affective effects can be collected. Also, during the actual scan, the user can be monitored taking the determined user profile into account.

As yet another example, some tests may be run to find personality traits that can be used to determine how the relation between equipment sounds and patient experience content 25 can be computed. Specifically, patients 4 may be classified as sensitive or insensitive to external stimuli.

As yet another example, the monitored environment sounds may be rendered into a waveform. Said waveform may contain transitions and fragments. The fragments, which may have a length on the order of minutes, have a clear periodic sound of various beats per minute (BPMs), one per fragment. The transitions are short (e.g., on the order of seconds) and may contain small fragments of various sounds. The loudness of a medical imaging exam sequence may also vary. Pitch wise, the medical imaging exam sequence is not musical, most of the 'notes' are polyphonic and off, hence the method uses mostly the rhythm and loudness of the equipment sounds.

As yet another example, auditory content may be based on musical segments (riffs, melodies, percussions), i.e., musical loops, in MIDI format, making it easier to time flex them for matching desired BPM. Each of these segments can be annotated from style/feel perspective with multiple labels: rock, pop, dance, mystical, dreamy, punchy, sporty etc. These annotations are handy when combining these segments with each other, and also with the visual content in other embodiments. From the point of view of masking off-pitch equipment sounds, it makes sense to apply various modulation effects to these segments.

Some examples of congruent and synchronized character animations in relation to environment sounds are: one or more characters dancing, walking, running, hammering, brooming, to the rhythmic equipment sounds. Other examples of congruent and synchronized character animations are characters acting upon the equipment sounds, by for example ducking or jumping over obstacles, factory machines, or any other scene elements, or responding by certain postures (e.g., hands to the ears or standing stiff) or motions (e.g., shaking). In addition to the characters moving in accordance with the sound, objects in the animation may also rhythmically move. For example, clocks, a metal plate moving with wind, a shaking ice drink and so. Special attention may be paid to specific moments in or between image sequence(s). For example, the moments where the characteristics of the equipment sounds change more predominantly and can significantly influence the patient 4. For instance, to shift the attention of the patient 4 away from the equipment sound, the start of a next sequence may be aligned with special visual events such as a rocket taking off, plane landing, truck starting to move, and so on.

In a specific example relating to a medical imaging procedure performed by a medical imaging device, the patient experience content may be generated as follows: an imaging protocol is used to estimate the start and end times of each sound. Imaging protocols describe the type, order, and parameters of different sequences. Using these planning documents, the sound generation features are estimated. These features include, start time and end time of each sequence relative to the start of imaging process, start times, end times, and duration of different sounds per sequence, indicators showing if the sound is starting, continuing, or ending. Then, (pre-)recorded sequence sounds are used to extract sound features (e.g. tempo, beat, frequency,duration, etc.). In this step, signal processing is applied to extract relevant sound features of an equipment sound database. Of course, only the sounds corresponding to the planned imaging sequences are processed. In an optional step, patient hearing information (or a head transfer function) is used to calculate the patient specific sound perception related features (e.g., loudness). The patient specific head transfer function (HRTF) is a set of acoustic filters that describe the unique way the sound waves are modified as they travel to the patient's 4 eardrums. HRTF can enhance the listening experience by providing more accurate spatialization and localization of sound sources. The need for HRTF arises because each patient 4 has a slightly different head size and shape, torso, and outer ears. In addition, other acoustics related information related to the room, on the positioning of the medical imaging device, and patient's 4 head within the room may be computed. All of these acoustic functions (filters) can be applied to improve the sound perception of the patient 4. In a further step, the patient experience content theme is chosen and the objects (including characters) are matched to imaging sequences sound generation and sound signal features. For this, a sub-group of patient experience content themes is selected. The selection is done based on the sound (signal and generation) properties. For example, only themes with characters, objects, or stories suitable for motion related animations are selected. Then, a sub-group is selected based on the library of available or desired animations. For example, only the themes that can include the matching animations are selected or only the theme that matches the previously shown animation is selected. It is to be noted that the subgroup selection may be also done by selecting the first subgroup based on animations and the second subgroup based on sound. Alternatively, the selection of suitable themes can be done by simultaneously using both sound features and animation options. For example, the matching is done so that a story is told, where the movement of characters matches the story (e.g., a rocket taking off, a shaker moves in the rocket, characters dance on the moon, the rocket lands, etc.). Alternatively, themes or storyline elements are selected based on the equipment sound characteristics. As an example, a first animated character is on his way to a second animated character. In a loud sequence, the first animated character comes along a construction side, in a sequence with many different kinds of sounds, the second animated character jumps into a pile of gold. Another option is to select a theme based on the character of the equipment sounds. For example, in a not loud but staccato sequence a rainforest, while in a very loud sequence a thunderstorm. Then, theme elements are selected for animation generation. For the selected theme, the specific characters, objects, visuals, lights, etc. that can be animated are determined. Also, the way of said animation is determined. Sound features such as pitch, loudness, energy in specific frequency bands, tempo, beat duration, etc., are used to modify the characters movements to match these features in a visual manner. For instance, if a video sequence depicts some periodic movements (walking, jumping, etc.), that periodicity may be extracted from plotting a light intensity curve (sum of all pixels luminance values in a frame, or pixels of segmented moving objects/characters). That curve can be used to determine a periodicity of the video fragment. Further, time flex (delay or speedup) may be applied to the video in accordance with scanner sound periodicity using luminance peaks as anchors. That will require to re-render the video sequence to match it with the scanner tempo. Further, a real-time adaptation (modification) of the animations is performed. At this stage, the state 20 of the patient 4 may also be monitored. In addition, the real-time sounds are monitored. These are used to change the animations if needed. For example, real-time sounds can be used to improve the time alignment between animations and sounds, and the state 20 of the patient 4 may be used to determine if other types of animations and themes should be used.

As yet another example, a patient 4 response may be continuously monitored, e.g., facial expressions, heart rate (variability), muscle tension and/or breathing rate captured by a camera, e.g., an in-bore camera, or by the patient grip holding a communication button. The patient experience content is adapted accordingly, e.g., by stopping, making it non-synchronized, increasing the synchronization (e.g., in addition to hands, legs of a character may also start moving in synchrony, the number of characters may increase, lights and video may become synchronous). A strength of a synchronization related metric may be calculated, and may be linked to the physiological state of the patient 4. This is used as a means of monitoring the effectiveness of the adaptations. This information may be stored in a theme database as meta-information.

As yet another example, an automatic annotation of an equipment sound track may be performed. For this, the equipment sound track may be divided into segments: transitions for parts where the medical imaging device is preparing for a new scanning mode, and fragments where the actual medical imaging is happening. Per fragment, the BPM of the equipment sound are determined. A story line may then be linked to a video sequence, yet can also be done separately having intro, verse, chorus, bridge, etc. It may be beneficial to use the patient's 4 preferences in music, and/or anticipate the patient's 4 state of mind and adapt the story accordingly, e.g., replace a next `verse' if the original one does not fit the state 20 of the patient 4 anymore. Once the equipment sound track is divided into segments, audio segments and/or recordings that are suitable for and/or match the equipment sounds BPM wise may be selected, such that their descriptions match the story line. To automate machining, story line parts may be annotated with text describing its 'feel': uplifting, tragic, dark. In those sorts of terms, the textual descriptions may be matched with similar descriptions of audio segments from the repository. For the transitions, off tempo (or slow tempo) music or ambient sounds may be used. For fragments, where the medical imaging device has its own `beat', audio content with corresponding BPM (or its multiples) may be used. Once the story line outlines have been determined and the audio segments have been chosen, all this is brought into alignment with the equipment sound track BPMs. For that, audio fragments are timeflexed to the corresponding BPMs of the corresponding fragments of the equipment sound soundtrack, leaving transitions with off beat or slow tempo audio. From the audio perspective, it may be beneficial to have multiple instruments involved, representing a rhythm section and melodic one. Also, when aligning BPMs, variability in audio experience may be introduced, e.g., by using multiples of the original BPM given by the medical imaging device. Thus, for instance, when the medical imaging device produces 280 BPM sounds, the melody may rest on 140 BPM or 70 BPM.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measured cannot be used to advantage. Any reference signs in the claims should not be construed as limiting the scope.

### LIST OF REFERENCE SIGNS:

- 1: medical environment
- 2: medical device
- 3: patient table
- 4: patient
- 5: control unit
- 6: system for generating patient experience content
- 7: input unit
- 8: computing unit
- 9: output unit
- 10: microphone
- 11: screen
- 12: speaker
- 13: method
- 14: sound recognition
- 15: sound information
- 16: obtaining
- 17: library
- 18: generation
- 19: library of character animations
- 20: state
- 21: patient profile
- 22: addition
- 23: library
- 24: story or game engine
- 25: patient experience content

## Claims

1. System (6) for generating patient experience content (25) during a medical procedure performed by a medical device (2) in a medical environment (1), comprising
an input unit (7) adapted to receive sound information (15) about environment sounds produced in the medical environment (1), more particularly about equipment sounds produced by the medical device (2);
a computing unit (8) adapted to generate the patient experience content (25) relating to the environment sounds based on the sound information (15); and
an output unit (9) adapted to output the generated patient experience content (25).

2. System (6) according to claim 1, wherein the medical device (2) is a medical imaging device, e.g., a magnetic resonance imaging, MRI, device, a computed tomography, CT, imaging device, or an image guided therapy, IGT, imaging device, and/or a medical therapy device, e.g., an IGT therapy device, or a radiotherapy device.

3. System (6) according to claim 1 or 2, wherein the input unit (7) is adapted to receive the sound information (15) by
getting operation signals from the medical device (2) and obtaining sound information (15) based on the operation signals via a library (17) or processor translating operation signals into equipment sounds; and/or
monitoring the environment sounds via sound sensors, more particularly microphones (10), and applying sound recognition (14).

4. System (6) according to any one of claims 1 to 3, wherein the sound information (15) comprises
sound features, comprising at least one out of timing, rhythm, beats per minute, pitch, loudness, energy in specific frequency bands, tempo, beat duration, and spectral features; and/or
sound generation features such as start time, end time, duration of the sound, and sound continuance features.

5. System (6) according to any one of claims 1 to 4, wherein the patient experience content (25) comprises visual content, auditory content, tactile stimulus content, vestibular stimulus content, thermoception content and/or air flow content.

6. System (6) according to any one of claims 1 to 5, wherein the computing unit (8) is adapted to generate the patient experience content (25) such that it is congruent, synchronized and/or linked with the environment sounds.

7. System (6) according to any one of claims 1 to 6, wherein the patient experience content (25) relates to at least one motion related element, more particularly an animated character and/or changing theme features.

8. System (6) according to any one of claims 1 to 7, wherein the computing unit (8) comprises a character and/or object animation rendering engine, more particularly comprising a library (19) and/or function translating sound information (15) into character and/or object animations.

9. System (6) according to any one of claims 1 to 8, wherein the computing unit (8) is further adapted to select a patient experience content theme, based on, for example, a patient profile (21) and/or available animation options for the patient experience content theme.

10. System (6) according to any one of claims 1 to 9, wherein the computing unit (8) is further adapted to include further visual content, auditory content, and/or game content in the patient experience content (25).

11. System (6) according to any one of claims 5 to 10, wherein the computing unit (8) is adapted to generate the auditory content and/or visual content by combining audio segments and/or video segments from an archive, based, for example, on beats per minute, volume, and/or duration.

12. Medical apparatus comprising
a medical device (2);
a system (6) for generating patient experience content (25) according to any one of claims 1 to 11; and
at least one output device (11; 12) adapted to receive the generated patient experience content (25) and provide the patient experience content (25) to a patient (4).

13. Method (13) for generating patient experience content (25) during a medical procedure performed by a medical device (2) in a medical environment (1), comprising the steps of:
receiving sound information (15) about environment sounds produced in the medical environment (1), more particularly about equipment sounds produced by the medical device (2);
generating the patient experience content (25) relating to the environment sounds based on the sound information (15);
outputting the generated patient experience content (25); and
providing the patient experience content (25) to a patient (4).

14. Method (13) according to claim 13, further comprising:
monitoring an effect of the patient experience content (25) onto a state (20), more particularly a mental state and/or a physiological state, of the patient (4);
wherein the patient experience content (25) is generated also based on the monitored effect onto the state (20) of the patient (4).

15. Method (13) according to claim 13 or 14, wherein the patient experience content (25) is generated also based on a patient profile (21).
